# EUROPÄISCHE PATENTANMELDUNG

(11) **EP 2 633 807 A1**
(43) Veröffentlichungstag der Anmeldung: **04.09.2013**
(21) Anmeldenummer: 13157147.3
(22) Anmeldetag: 28.02.2013
(51) Int. Cl.: A61B 5/00

(54) **Fixiergurt für die veterinärmedizinische Diagnostik bei Großtieren**

(30) Priorität: 02.03.2012 DE 202012100735 U
(71) Anmelder: FBN - Leibniz-Institut für Nutztierbiologie, 18196 Dummerstorf (DE)
(72) Erfinder: Hacke, Sandra, 19243 Döbbersen (DE); Pilz, Kerstin, 18069 Rostock (DE)
(74) Vertreter: Wablat Lange Karthaus

(57) **Zusammenfassung**

Die vorliegende Erfindung betrifft einen Fixiergurt für die veterinärmedizinische Diagnostik bei Großtieren mit einer Innenseite und einer Außenseite, wobei die Innenseite im Gebrauchszustand auf dem Fell des zu untersuchenden Großtieres aufliegt, mit einer langgestreckten Ausdehnung in einer Längsrichtung und einer Breite in einer Querrichtung, einen Gurt aus reißfestem Material und mindestens eine Schließvorrichtung umfassend, sowie einen veterinärmedizinischen Messgürtel zur Messung der Herzfrequenzvariabilität von Großtieren mit einer Innenseite und einer Außenseite, wobei die Innenseite im Gebrauchszustand auf dem Fell des zu untersuchenden Großtieres aufliegt, mit einer langgestreckten Ausdehnung in einer Längsrichtung und einer Breite in einer Querrichtung, einen Gurt aus reißfesten Material, mindestens eine Schließvorrichtung und ein Einlegeband mit mindestens zwei diagnostischen Sensoren umfassend, wobei die diagnostischen Sensoren des Einlegebandes elektrisch leitend im Einlegeband miteinander verbunden sind und zwischen den Sensoren eine Tasche für eine anschließbare Steuer- und/oder Sendeeinheit eingearbeitet ist.

## Beschreibung

Die vorliegende Erfindung betrifft einen Fixiergurt für die veterinärmedizinische Diagnostik bei Großtieren nach dem Oberbegriff von Anspruch 1 und einen veterinärmedizinischen Messgürtel nach dem Oberbegriff von Anspruch 10.

Das Herz erzeugt elektrische Signale, die auf der Körperoberfläche, z.B. der Haut oder dem Fell von Tieren, gemessen werden können. Mit Hilfe von Elektroden, welche die Signale aufnehmen, und einem Sender, welcher die Signale von den Elektroden aufnimmt und weiterübermittelt, können die elektrischen Signale auf eine Auswerteeinheit übertragen werden. Eine solche Auswerteeinheit ist zumeist ein Computer.

Anhand der gemessenen elektrischen Signale kann die Herzfrequenz bzw. Herzschlagfrequenz ermittelt werden. Die Herzfrequenz ist die Anzahl der Herzschläge pro Minute. Sie wird angegeben in min⁻¹ oder als bpm (beats per minute). Die Herzschlagfrequenz ist u.a. abhängig von der Belastung, vom Alter und von der körperlichen Verfassung des jeweiligen zu untersuchenden Individuums.

Als Herzfrequenzvariabilität (englisch Heart Rate Variability, HRV) wird die Fähigkeit eines Organismus (Mensch, Säugetier) bezeichnet, die Frequenz des Herzrhythmus zu verändern. Auch im Ruhezustand treten spontan Veränderungen des zeitlichen Abstandes zwischen zwei Herzschlägen auf. Unter der HRV versteht man dementsprechend Schwankungen der Herzfrequenz über einen kürzeren oder längeren Messzeitraum bei einer Analyse von Herzschlag zu Herzschlag. Die Untersuchung der einzelnen Frequenzspektren erfolgt über verschiedene mathematische Verfahren. Methodisch kann die HRV von Herzschlag zu Herzschlag über Zeiträume von Minuten bis zu Stunden mittels Langzeit-EKG gemessen werden (H. Löllgen, Deutsches Ärzteblatt, 1999, 96, 31-32, 45-48). Die HRV kann als leistungsdiagnostische Kenngröße, als Kontrollparameter der Beanspruchung und als Steuerparameter der Belastungsintensität herangezogen werden.

Messgurte oder Messbänder zur veterinärmedizinischen Untersuchung der Herzfrequenz von Großtieren sind in der Technik bekannt, so gibt es beispielsweise einen Messgurt für den Reitsport, der um die Brust und den Rücken (Widerrist) eines Pferds gelegt werden kann (Equine Gurt, Firma Polar). Es handelt sich hierbei um ein Textilband, welches zwei Elektroden enthält, an welche ein kabelloser Sender angeschlossen wird (Equine WearLink®-Sender, Firma Polar). Das Band ist im Bereich außerhalb der Elektroden einstellbar und elastisch ausgebildet, zwischen den Elektroden ist das Band hingegen starr und nicht einstellbar. Der Sender übermittelt die Informationen zu den gemessenen elektrischen Signalen an eine Empfänger- bzw. Auswerteeinheit, wobei es sich zumeist um einen Computer handelt. Im Spezialfall ist dieser Computer in Form einer Armbanduhr ausgebildet (z.B. Equine RS800CX Trainingscomputer, Firma Polar). Dieses System wird nachfolgend als das Polar-System bezeichnet.

Die Messung der Herzfrequenz bzw. der HRV ist äußerst sensibel. Die Elektroden müssen immer an der richtigen Stelle anliegen, um durchgängig genaue Messergebnisse erhalten zu können. Die verrutschfreie Anbringung der Elektroden am Körper eines Großtieres ist für die Ergebnissicherheit und Zuverlässigkeit der erhobenen Daten von großer Bedeutung. Aufgrund der unterschiedlichen Morphologie von Großtieren ergeben sich stärkere Abweichungen, beispielsweise hinsichtlich des Körperumfangs. Hinzu kommt, dass das angelegte System das Tier nicht in seiner natürlichen Bewegung einschränken und kein Unwohlsein verursachen darf, da dies zu einer Verfälschung der Messergebnisse führen würde. Das System muss Langzeitmessungen unter unterschiedlichen Bedingungen wie bei Stall- oder Freilandhaltung erlauben.

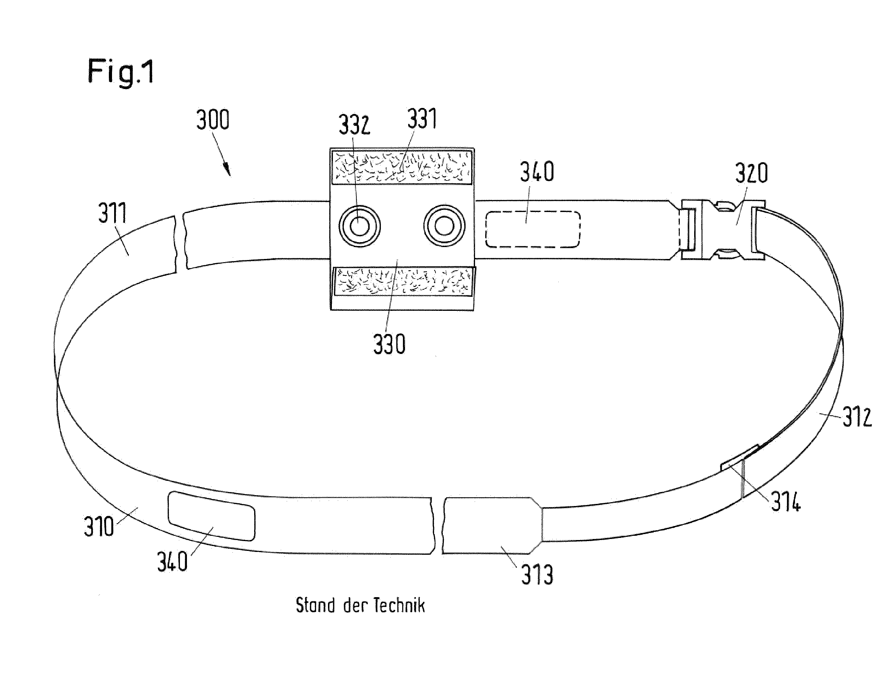

Nachteilig bei dem bekannten Polar-System ist dessen nahezu starrer Aufbau insbesondere im Bereich zwischen den beiden Elektroden sowie die schlechte Fixierbarkeit (vgl. Figur 1). Es ermöglicht zwar im Vergleich zu vorher bekannten Systemen eine bessere Anpassung an den Tierkörper und weist eine längere Elektrodenverbindung auf, neigt allerdings schnell zum Verrutschen, wodurch sich eine Unterbrechung der Herzfrequenzmessung ergibt. Weiterhin ist der Abstand zwischen den Elektroden nicht variabel einstellbar, so dass eine Anpassung an verschiedene Morphologien, d. h. Körperumfänge bzw. Staturen, nicht möglich ist. Dies ist insbesondere bei Großtieren wie beispielsweise Rindern oder Pferden relevant, welche in ihren Körperproportionen große Varianzen aufweisen (z.B. Fohlen, Pony, Kleinpferd, Großpferd oder Kalb, Rind).

Das technische Problem war somit die Bereitstellung eines für die Herzvariabilitätsmessung geeigneten Messsystems, welches im Hinblick auf den Abstand der beiden diagnostischen Sensoren mit größerer Varianz an verschiedene Körperumfänge bzw. Staturen angepasst werden kann und welches eine bessere Fixierbarkeit auch bei unterschiedlichen Großtierarten aufweist.

Diese Aufgabe wurde mit einem Fixiergurt für die veterinärmedizinische Diagnostik bei Großtieren gemäß Anspruch 1 sowie mit einem veterinärmedizinischen Messgürtel zur Messung der Herzfrequenzvariabilität von Großtieren gemäß Anspruch 10 gelöst. Weitere bevorzugte Ausgestaltungen und Weiterbildungen des Fixiergurts für die veterinärmedizinische Diagnostik bei Großtieren ergeben sich aus den abhängigen Ansprüchen.

Großtiere sind beispielsweise Rinder, Pferde, Schweine, Esel etc., wobei vorzugsweise der Brustkorbumfang mindestens einen Meter beträgt. Nachfolgend wird mehrfach auf Pferde und Rinder als bevorzugte Großtiere eingegangen, die Erfindung ist aber nicht auf diese Tiere beschränkt. Gleichwohl anwendbar ist die Erfindung bei anderen Großtieren, beispielsweise ZootierenM/ildtieren wie Giraffe, Kamel, Dromedar und Elch.

In anderen Worten wurde die Aufgabe mit einem Fixiergurt für die veterinärmedizinische Diagnostik bei Großtieren gelöst, welcher eine Innenseite und eine Außenseite aufweist, wobei die Innenseite im Gebrauchszustand auf dem Fell des zu untersuchenden Großtieres aufliegt, mit einer langgestreckten Ausdehnung in einer Längsrichtung und einer Breite in einer Querrichtung, einen Gurt aus reißfestem Material und mindestens eine Schließvorrichtung umfassend und wobei außerdem an der Innenseite eine oder mehrere Haltevorrichtungen für mindestens zwei diagnostische Sensoren vorgesehen sind, welche geeignet ist bzw. sind, die diagnostische Sensoren in einem variabel einstellbaren Abstand zueinander aufzunehmen.

Vorzugsweise ist das Material des Gurtes zumindest im Bereich zwischen den diagnostischen Sensoren elastisch.

Bevorzugt ist, dass die Haltevorrichtungen geeignet sind, handelsübliche diagnostischen Sensoren aufzunehmen, wobei die diagnostischen Sensoren vorzugsweise an einem Einlegeband angeordnet sind, in dem eine elektrisch leitende Verbindung zwischen den Sensoren und eine Tasche für eine anschließbare Steuer- und/oder Sendeeinheit eingearbeitet sind und wobei das Einlegeband besonders vorzugsweise eine Breite aufweist, die geringer als die Breite des Fixiergurtes ist.

Die diagnostischen Sensoren des Einlegebandes sind ferner bevorzugt als Elektroden ausgestaltet, die vorzugsweise für eine Herzfrequenzvariabilitätsmessung geeignet sind.

Unter einem solchen Einlegeband mit daran angeordneten diagnostischen Sensoren sind beispielsweise die verschiedenen Textil-Elektroden der Firma Polar zu verstehen (z.B. Equine Textil-Elektroden für WearLink®+ Sender), bei denen die Elektroden jeweils an den Enden eines textilen Bandes zu finden sind. An dieses Einlegeband ist die entsprechende Steuer- und/oder Sendeeinheit anzukoppeln. Als Steuer-/Sendeeinheit kommen hierfür der Polar Equine T54H Textil-Sender (codiert) oder der T56H Textil-Sender W.I.N.D. der Firma Polar in Frage. Dieses System war bisher von der Anlegung an Pferden derart gedacht, dass die Plus-Elektrode zuerst unter eine Satteldecke gesteckt und anschließend die Minus-Elektrode am Sattelgurt befestigt wurde. Der Sender wurde am Sattel fixiert. Allerdings ist die vorliegende Erfindung nicht auf die Verwendung dieser Polar-Komponenten beschränkt, vielmehr können auch andere Alternativen als Einlegeband, diagnostische Sensoren bzw. Steuer-/Sendeeinheit eingesetzt werden.

Die an der Innenseite vorgesehene(n) eine oder mehrere Haltevorrichtungen für mindestens zwei diagnostische Sensoren, welche geeignet ist bzw. sind, die diagnostische Sensoren in einem variabel einstellbaren Abstand zueinander aufzunehmen, ermöglichen die Anpassung an variable Morphologien. Beispielsweise können bei Tieren mit einem größeren Körperumfang die diagnostischen Sensoren/Elektroden in einem größeren Abstand zueinander positioniert werden, als dies bei Tieren mit geringerem Körperumfang erforderlich wäre. Bei Tieren mit geringerem Körperumfang "überflüssige" Bandbereiche des Einlegebandes können variabel aufbewahrt werden, wobei sie natürlich auch frei/unfixiert verbleiben können.

Besonders bevorzugt ist, dass der Fixiergurt an seiner Innenseite innerhalb des Bereichs der Haltevorrichtung(en) zusätzlich mindestens eine in Längsrichtung offene Tasche aufweist, in welche vorzugsweise neben der Tasche des Einlegebandes mit der Steuer- und/oder Sendeeinheit für die veterinärmedizinische Diagnostik auch nicht entfaltete Bandbereiche des Einlegebandes aufnehmbar sind. Diese zusätzliche Tasche ermöglicht es, "überflüssige" Bandbereiche des Einlegebandes, inklusive der die Steuer- und/oder Sendeeinheit enthaltenden Tasche des Einlegebandes fest aufzubewahren. Diese überflüssigen Bandbereiche können in jedweder Form in die Tasche eingebracht werden, beispielsweise können sie mehrschichtig gefaltet werden.

Es kann außerdem vorgesehen sein, dass als Haltevorrichtungen am Fixiergurt für die diagnostischen Sensoren Klettverschlüsse, Halteriemen und/oder Druckknopfverschlüsse vorgesehen sind. Bevorzugte Ausführungsformen dieser Haltevorrichtungen werden nachfolgend bei genauer Beschreibung der Figuren dargelegt.

Die Haltevorrichtungen für die diagnostischen Sensoren sind insgesamt selbst vorzugsweise als in Längsrichtung offene Taschen ausgebildet. Für eine bessere Anpressung der diagnostischen Sensoren an den Tierkörper können in diese offenen Taschen Unterlegmaterialien, beispielsweise handelsübliche Küchenschwämme, eingeschoben werden. Deren Einbringung führt zu einer Exponierung der diagnostischen Sensoren im Vergleich zur Ebene des Fixiergurtes und somit zu einer bei Anbringung verstärkten Anpressung.

Eine weitere Ausgestaltungsform sieht vor, dass die Schließvorrichtung des Fixiergurts Klettverschlüsse und/oder Riemen und Schnallen umfasst.

Außerdem kann es vorgesehen sein, dass der Fixiergurt zum Umschnallen um Bauch und Widerrist/Rücken des Großtieres ausgelegt ist. Bei Anwendung in der Praxis, vorzugsweise an Pferden und Rindern als Großtieren konnte gezeigt werden, dass der Gurt auch bei stark differenten Morphologien gut an die einzelnen Tiere adaptiert werden kann. Insbesondere können die Elektroden des Einlegebandes aufgrund des erfindungsgemäßen Fixiergurtes gut an geeigneten Stellen auf der Körperoberfläche des Tieres positioniert werden.

Die Kombination aus Fixiergurt und Einlegeband wird nachfolgend auch als Gurtsystem bzw. veterinärmedizinischer Messgürtel bezeichnet. Die Erfindung betrifft dementsprechend auch einen veterinärmedizinischen Messgürtel zur Messung der Herzfrequenzvariabilität von Großtieren mit einer Innenseite und einer Außenseite, wobei die Innenseite im Gebrauchszustand auf dem Fell des zu untersuchenden Großtieres aufliegt, mit einer langgestreckten Ausdehnung in einer Längsrichtung und einer Breite in einer Querrichtung, einen Gurt aus reißfesten Material, mindestens eine Schließvorrichtung und ein Einlegeband mit mindestens zwei diagnostischen Sensoren umfassend, wobei die diagnostischen Sensoren des Einlegebandes elektrisch leitend im Einlegeband miteinander verbunden sind und zwischen den Sensoren eine Tasche für eine anschließbare Steuer- und/oder Sendeeinheit eingearbeitet ist und wobei außerdem an der Innenseite eine oder mehrere Haltevorrichtungen für die diagnostische Sensoren des Einlegebandes vorgesehen sind, welche geeignet sind, die diagnostischen Sensoren in einem variabel einstellbaren Abstand zueinander aufzunehmen.

Das Gurtsystem erwies sich bei Anwendung in der Praxis an Großtieren, insbesondere an Rindern und Pferden, als gut geeignet. Es erlaubte die Adaptierung des Systems an unterschiedlichste Morphologien der jeweiligen Tiere, wobei insbesondere die Elektroden des Einlegebandes gut und sicher auf den jeweiligen Positionen auf der Körperoberfläche des Tieres positioniert werden konnten. Das Gurtsystem schränkte die Tiere nicht ihren natürlichen Bewegungen ein und verursachte kein Unwohlsein. Es erwies sich auch bei unterschiedlichsten Bedingungen wie beispielsweise Freiland- oder Stallhaltung als zur Messung gut geeignet. Auch Spezialmessungen, beispielsweise in einer Respirationskammer oder beim Transport ließen sich problemlos durchführen. Das Gurtsystem blieb ohne Verrutschen an der zu Beginn eingestellten Position am Tierkörper, so dass die Elektroden ebenfalls ohne Verrutschen in ihren vorbestimmten Positionen verblieben und eine unterbrechungsfreie Messung möglich war.

Insbesondere bei Freilandmessungen war zu beachten, dass für eine störungsfreie Übermittlung der Messwerte ein gewisser Maximalabstand zwischen Sende-/Steuereinheit und Empfänger- bzw. Auswerteeinheit, wobei es sich zumeist um einen Computer handelt, nicht überschritten werden sollte. Beispielhaft ist die Empfängereinheit in Form einer Armbanduhr ausgebildet (z.B. Equine RS800CX Trainingscomputer, Firma Polar). Um hier unnötige Beeinträchtigung des Tieres, wie ein "Hinterherlaufen" zu vermeiden, weist der Fixiergurt in einer besonders bevorzugten Ausgestaltungsform an seiner Außenseite eine weitere Tasche für den Empfänger/die Uhr auf. In dieser Tasche kann der Empfänger direkt am Tier positioniert werden, wodurch der Abstand zwischen Sende- und Empfängereinheit fixiert wird. Nach beendeter Messung kann der Empfänger dann entnommen werden und die Messwerte können auf einen weiteren externen Computer übertragen werden.

Mit diesem Gurtsystem kann die Herzfrequenz bzw. HRV auch bei Langzeitmessungen von über 24 h und mehr durchgeführt werden, wobei unterschiedliche Versuchsdurchführungen (Freiland, Stall, Respirationskammer, Transport) ermöglicht werden. Mit Hilfe der HRV-Messung konnten Aussagen zum Energiestoffwechsel, sowie dem Verhalten getroffen werden und inwieweit das Tier unter Stress steht, da die Auswertung der Messdaten die Aktivität des vegetativen Nervensystems widerspiegelt (Aktivität des Sympatikus und Parasympathikus). Die Herzfrequenz hat eine starke Korrelation zum Energiestoffwechsel, wodurch auch eine Korrelation zwischen HRV und dem Energiestoffwechsel gegeben ist. Anhand der HRV-Messung, wird untersucht, ob ein Tier in die negative Energiebilanz kommt und ob somit Stoffwechselerkrankungen frühzeitig vorhersehbar sind. Weiterhin wird untersucht, wie stark und wie lange beispielsweise eine Kuh um die Abkalbung herum Stressmomente hat und wie schnell sie sich an diese Situation anpasst bzw. nach dem Abkalben wieder regenerieren kann.

Mit der HRV-Messung in der Respirationskammer bei beispielsweise Rindern ist eine vielfältige Untersuchung des gesamten Tieres möglich, die bei keiner anderen Messungssituation im Freiland oder Stall gegeben ist. Es herrschen optimale Bedingungen, um zum Beispiel andere Faktoren im Stall oder Freiland (Hitze- oder Kältestress, Lärm, Unruhe in der Gruppe), die beim Tier einwirken konnten, auszuschließen. Die Tiere werden hierbei u.a. mit Kameras beobachtet und die Aktivität des Tieres mit Sensoren gemessen. Die Futteraufnahme, der Sauerstoffverbrauch und die Kohlenstoffdioxid- und Methanproduktion kann ganz genau ermittelt werden, wodurch eine Berechnung des Energiestoffwechsels ermöglicht wird. Durch eine kontinuierliche Blutentnahme des Tieres (außerhalb der Kammer durch einen Katheter mit Verlängerung), kann auch eine Verbindung zu den hormonellen und biochemischen Prozessen bei unterschiedlichen Filterungsbedingungen, beispielsweise vor und nach der Abkalbung bei einer Kuh untersucht werden. Die Auswertung der HRV ist durch diese vielfältige Untersuchung in der Respirationskammer viel exakter und weitreichender interpretierbar. Diese Erkenntnisse könnten auch hilfreich für die Auswertung und Beurteilung der HRV im Freiland oder im Stall sein.

Weitere Eigenschaften, Merkmale und Vorteile der Erfindung werden nachfolgend anhand von Figuren zu bevorzugten Ausführungsformen des Fixiergurts erläutert.

### Im Einzelnen zeigt

- Fig. 1: eine schematische perspektivische Darstellung des Standes der Technik, d.h. einen Polar-Equine Gurt,
- Fig. 2: eine schematische Aufsicht der Außenseite des Fixiergurts nach einer Ausführungsform der Erfindung,
- Fig. 3A: eine schematische Aufsicht der Innenseite des Fixiergurts nach einer ersten Ausführungsform der Erfindung mit variabel anheftbaren Haltevorrichtungen für die diagnostischen Sensoren,
- Fig. 3B: eine schematische Aufsicht der Innenseite des Fixiergurts nach einer zweiten Ausführungsform der Erfindung mit Haltevorrichtungen für ein variables Anheften der diagnostischen Sensoren,
- Fig. 4A: eine schematische Aufsicht der Innenseite des Fixiergurts nach der ersten Ausführungsform der Erfindung mit an den variabel anheftbaren Haltevorrichtungen angebrachtem Einlegeband mit diagnostischen Sensoren,
- Fig. 4B: eine schematische Aufsicht der Innenseite des Fixiergurts nach der zweiten Ausführungsform der Erfindung mit an den Haltevorrichtungen (variables Anheften der diagnostischen Sensoren) angebrachtem Einlegeband mit diagnostischen Sensoren,
- Fig. 5A: eine schematische Aufsicht der Innenseite des Fixiergurts nach der ersten Ausführungsform der Erfindung mit an den variabel anheftbaren Haltevorrichtungen angebrachtem Einlegeband mit diagnostischen Sensoren, wobei der in eine in Längsrichtung offene Tasche aufnehmbare Teil des Einlegebandes hervorgehoben ist,
- Fig. 5B: eine schematische Aufsicht der Innenseite des Fixiergurts nach der zweiten Ausführungsform der Erfindung mit an den Haltevorrichtungen (variables Anheften der diagnostischen Sensoren) angebrachtem Einlegeband mit diagnostischen Sensoren, wobei der in eine in Längsrichtung offene Tasche aufnehmbare Teil des Einlegebandes hervorgehoben ist,
- Fig. 6A: eine schematische Aufsicht eines vergrößerten Ausschnitts der Innenseite des Fixiergurts aus Fig. 5A, wobei nur der links gezeigte diagnostische Sensor bereits an der voreingestellten Haltevorrichtung angebracht ist,
- Fig. 6B: eine schematische Aufsicht eines vergrößerten Ausschnitts der Innenseite des Fixiergurts aus Fig. 5B, wobei nur der links gezeigte diagnostische Sensor bereits voreingestellt an der Haltevorrichtung angebracht ist,
- Fig. 7A: eine schematische perspektivische Darstellung des geschlossenen Fixiergurts nach der ersten Ausführungsform der Erfindung mitangebrachtem Einlegeband,
- Fig. 7B: eine schematische perspektivische Darstellung des geschlossenen Fixiergurts nach der zweiten Ausführungsform der Erfindung mit angebrachtem Einlegeband.

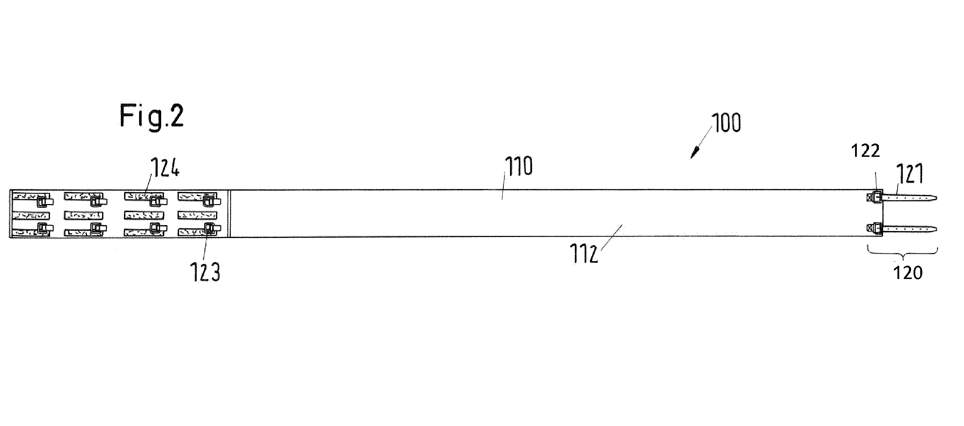

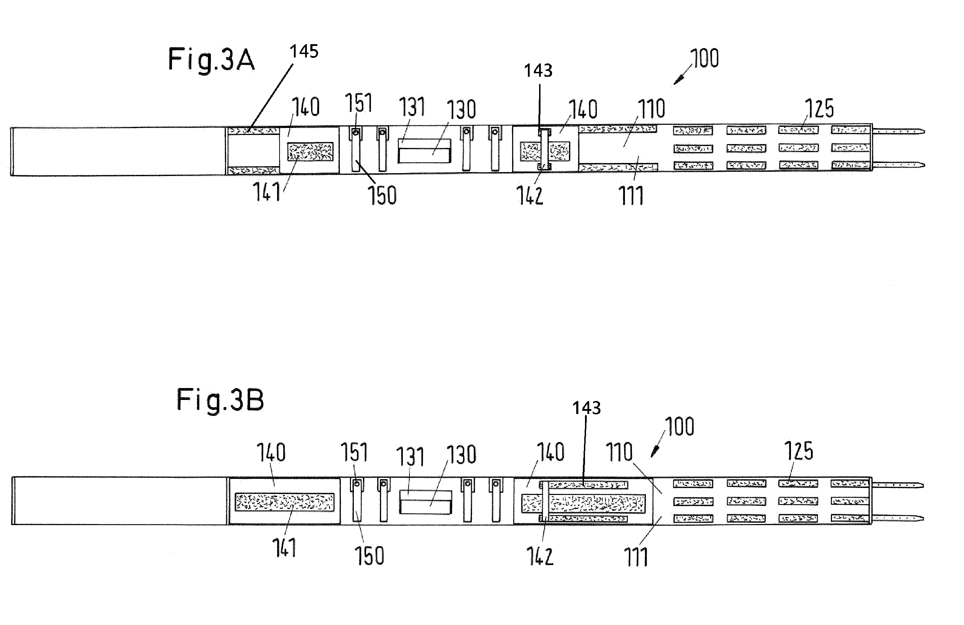

In der folgenden ausführlichen Beschreibung der Figuren werden Ausführungsformen der Erfindung näher erläutert. Insbesondere ist eine Kombination der in Fig. 2 dargestellten Ausführungsform mit der ersten Ausführungsform der Erfindung aus den Figuren 3A, 4A, 5A, 6A und 7A oder der zweiten Ausführungsform der Erfindung aus den Figuren 3B, 4B, 5B, 6B und 7B vorgesehen.

Fig. 1 zeigt einen Polar-Equine Gurt gemäß dem Stand der Technik in geschlossener Form, wobei der Gurt zur Messung der HVR 300 eine Innenseite 310 und eine Außenseite 311 und einen flexiblen einstellbaren Bereich 312 sowie einen rigiden Bereich 313 mit den Elektrodenbereichen 340 aufweist. Eine Verlängerungseinrichtung des flexiblen Bereiches 314 ermöglicht eine Einstellbarkeit des Umfangs, wobei der Abstand der Elektroden im Bereich 312 stets konstant bleibt. Geschlossen wird der Messgurt über einen Steckverschluss 320. Der Gurt weist eine Tasche 330 für eine anschließbare Steuer- und/oder Sendeeinheit auf, welche mit einem Klettverschluss 331 zu schließen ist. Weiterhin sind Anschlüsse/Druckknöpfe 332 für eine anschließbare Steuer- und/oder Sendeeinheit vorhanden.

In Fig. 2 ist die Außenseite 112 eines Fixiergurts 100 nach einer Ausführungsform der Erfindung dargestellt, welcher einen Gurt 110 umfasst, wobei an einem ersten auf der rechten Seite dargestellten Ende in Längsrichtung des Fixiergurtes 100 Schließvorrichtungen120 angebracht sind, die wie hier gezeigt aus Riemengurten 121 und Riemenschnallen 122 bestehen und wobei sich in einem auf der linken Seite dargestellten Bereich am entgegengesetzten Ende in Längsrichtung des Fixiergurtes 100 korrespondierende Riemenumlenkschnallen 123 befinden. Im Bereich des Fixiergurtes 100 mit den Riemenumlenkschnallen 123 sind weiterhin für die bessere Fixierung des Fixiergurts Klettbänder 124 vorgesehen. Die Klettbänder können beispielsweise als Haken- oder Pilzband ausgelegt sein, wenn an der Innenseite des Fixiergurts (nicht in Fig. 2 dargestellt) ein hierzu korrespondierendes Velours-oder Flauschband vorliegt. Die beiden Klettband-Typen können natürlich auch miteinander vertauscht vorliegen.

Im Folgenden beziehen sich die mit A indizierten Figuren (3A, 4A, 5A, 6A und 7A) stets auf die erste Ausführungsform der Erfindung, bei der die Haltevorrichtungen 140 vollständig als Einheit am Fixiergurt variabel positionierbar sind. Die mit B markierten Figuren (3B, 4B, 5B, 6B und 7B) beziehen sich auf die zweite Ausführungsform der Erfindung, bei der die Haltevorrichtung 140 fest an der Innenseite 111 des Fixiergurts angebracht sind, aber ihrerseits eine variable Anordnung der diagnostischen Sensoren 220 ermöglichen.

Fig. 3A veranschaulicht die Innenseite 111 des Fixiergurts 100 nach der ersten Ausführungsform der Erfindung, welcher einen Gurt 110 mit Haltevorrichtungen 140 für die diagnostischen Sensoren wie beispielsweise Elektroden eines Einlegebandes umfasst. Bei der in Fig. 3A dargestellten Ausführungsform des Fixiergurts kann die gesamte Haltevorrichtung 140 des Fixiergurts 100 mittels längeren Klettbändern 145 voreingestellt werden. Wird für eine Messung aufgrund der Morphologie des zu untersuchenden Tieres ein größerer Abstand der Elektroden benötigt, wird zumindest eine der Haltevorrichtungen 140 vollständig vom Fixiergurt 100 abgenommen und an den Klettbändern 145 bedarfsgerecht umgesetzt. Die Klettbänder 125 in einem Bereich am rechts dargestellten Ende des Fixiergurtes 100 sind beispielsweise als (Velours- oder Flauschbänder) ausgeführt. Die beidseitig vorhandenen Haltevorrichtungen 140 sind jeweils mit Klettbändern 141 versehen, auf denen die diagnostischen Sensoren fixiert werden können. Zusätzlich kann wie in der Fig. 3A gezeigt zumindest einseitig ein zusätzlicher Halteriemen 142 vorhanden sein, welcher-aufgrund einer ggf. vorhandenen Lasche auf der Rückseite eines diagnostischen Sensors-, durch diese Lasche gezogen werden kann und somit zur zusätzlichen Fixierung des Sensors dient. Der Halteriemen 142 wird im gezeigten Beispiel mittels Klettband 143 fixiert. Bei der in Fig. 3A dargestellten ersten Ausführungsform des Fixiergurts 100 sind die Haltevorrichtungen 140 somit mittels der Klettbänder 145, in einem größeren vorgegebenen Bereich, was Anpassungen an die unterschiedlichen zu untersuchenden Tiere erlaubt, variabel an der Innenseite 111 des Fixiergurtes positionierbar. Falls anstatt von Klettbändern 145 eine andere Fixierung der Haltevorrichtungen 140 am Fixiergurt 100 vorgesehen ist, ist auch eine anderweite Verschiebung der Haltevorrichtungen 140 am Fixiergurt 100 denkbar. Annähernd mittig an der Innenseite 111 befindet sich eine in Längsrichtung offene Tasche 130, welche mittels Klettverschluss 131 verschließbar ist. Diese Tasche dient neben der Aufnahme einer Steuer-und/oder Sendeeinheit eines Einlegebandes auch der Arretierung von aufgrund der Positionierung der Sensoren (nicht in Fig. 3A gezeigt) nicht benötigten Bereichen des Einlegebandes selbst. Zusätzlich sind bei der gezeigten Ausführungsform Halteriemen 150 für das einzulegende Einlegeband vorgesehen, welche beispielsweise mit Druckknöpfen 151 verschließbar sind.

Anhand der Fig. 3B wird die Innenseite 111 des Fixiergurts 100 nach der zweiten Ausführungsform der Erfindung veranschaulicht, wobei der Fixiergurt 100 einen Gurt 110 mit Haltevorrichtungen 140 für die diagnostischen Sensoren 220 umfasst und wobei sich in einem Bereich am rechts dargestellten Ende des Fixiergurtes 100 Klettbänder 125 (Velours- oder Flauschband) befinden. Der wesentliche Unterschied zur in Fig. 3A gezeigten ersten Ausführungsform besteht darin, dass bei der hier gezeigten zweiten Ausführungsform der Erfindung die Haltevorrichtungen 140 nicht als ganzes an der Innenseite 111 des Fixiergurts 100 in einem größeren Bereich (s.o.) positionierbar und somit voreinstellbar sind, sondern dass die Haltevorrichtungen 140 selbst größer ausgebildet sind und einen größeren Arretierungsbereich 141 zur Montage der diagnostischen Sensoren (nicht in Fig. 3B gezeigt) aufweisen. Alle anderen Merkmale entsprechen denen der ersten Ausführungsform, sodass auf die entsprechenden Ausführungen zur Fig. 3A verwiesen wird.

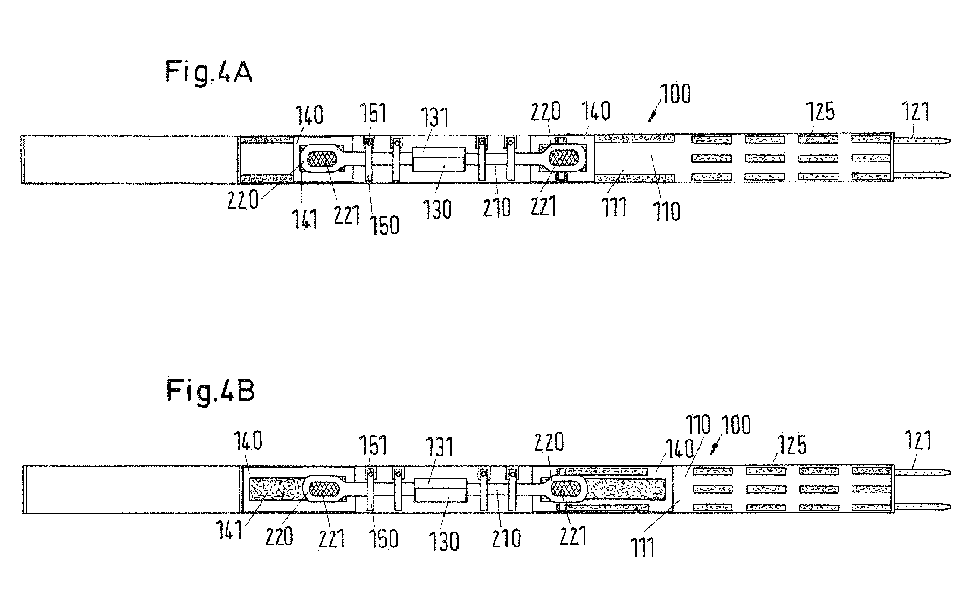

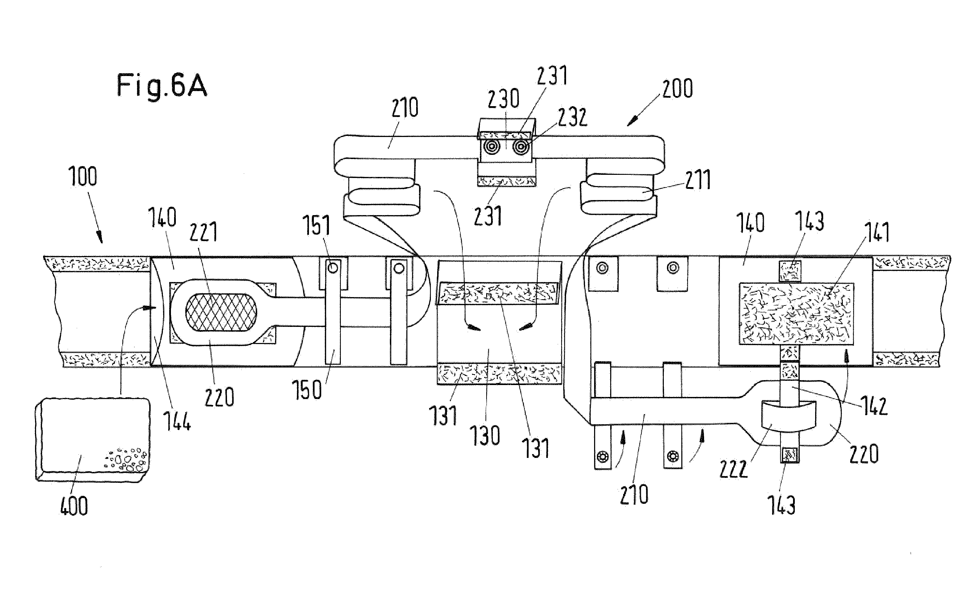

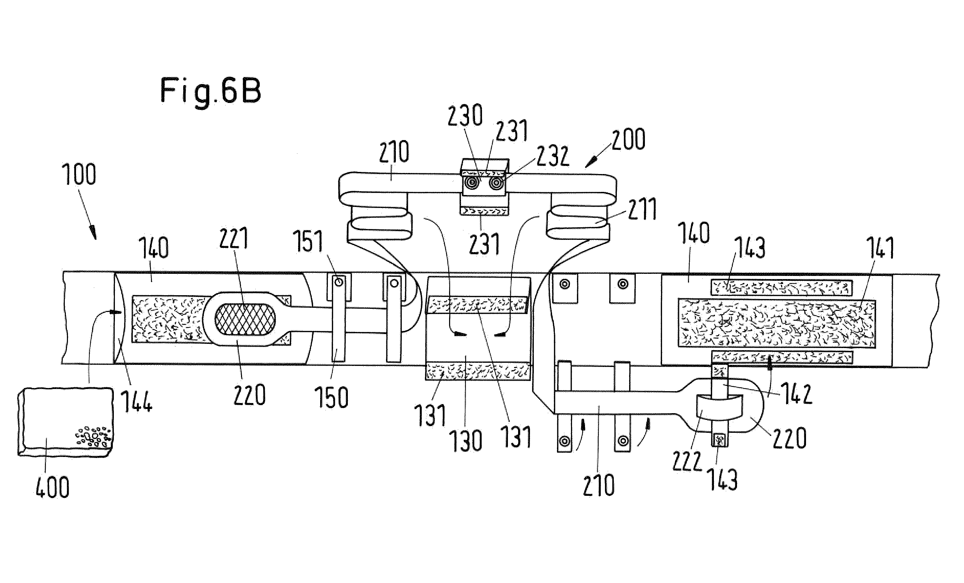

Die Fig. 4A und 4B zeigen jeweils die entsprechende erst bzw. zweite Ausführungsform der Erfindung mit an der Innenseite 111 des Fixiergurts 100 angebrachtem Einlegeband 200, welches entfaltete Bereiche 210 und nicht entfaltete Bereiche 211, die sich in der Tasche 130 befinden und daher nicht zu sehen sind, aufweist. Die an den Enden des Einlegebandes 200 befindlichen diagnostischen Sensoren 220, welche nach innen gewandte Messbereiche 221 aufweisen, sind auf den Haltevorrichtungen 140 mittels Klettbändern 141 bzw. Halteriemen 142 fixiert. Bei der ersten Ausführungsform in Fig. 4A sind die als Elektroden ausgeführten diagnostischen Sensoren 220 ungefähr mittig auf den Haltevorrichtungen 140 angebracht. Die Haltevorrichtungen 140 sind wiederum durch Klettverschlüsse am Fixiergurt 100 befestigt. Im in Fig. 4A gezeigten Beispiel sind die Haltevorrichtungen 140 mit dem kleinsten möglichen Abstand zueinander am Fixiergurt 100 angeordnet. Für größere Nutztiere kann der Abstand der diagnostischen Sensoren 220 durch Repositionieren der Haltevorrichtungen 140 vergrößert werden. Bei der zweiten Ausführungsform in Fig. 4B sind die als Elektroden ausgeführten diagnostischen Sensoren 220 fast mit dem kleinsten einstellbaren Abstand zueinander auf den Haltevorrichtungen 140 angebracht. Die Haltevorrichtungen 140 sind bei der zweiten Ausführungsform der Erfindung fest am Fixiergurt 100 angeordnet. Im in Fig. 4B gezeigten Beispiel kann der Abstand der Elektroden 220 zueinander vergrößert werden, indem eine oder beide Elektroden 220 weiter von der Tasche 130 entfernt an den Haltevorrichtungen 140 befestigt werden. Außerdem ist den Fig. 4A und 4B zu entnehmen, dass die links und rechts gezeigte Elektrode 220 (linke und rechte Elektrode 220) jeweils auf unterschiedliche Weise an der Haltevorrichtung 140 fixiert sind. Während die linken Elektroden 220 im gezeigten Beispiel an den linken Haltevorrichtungen 140 angeklettet sind, sind die rechten Elektroden 220 mittels von Halteriemen 142 mit Klettverschluss 143 (vgl. Fig. 6A bzw. 6B) an den rechten Haltevorrichtungen 140 des Fixiergurts 100 befestigt.

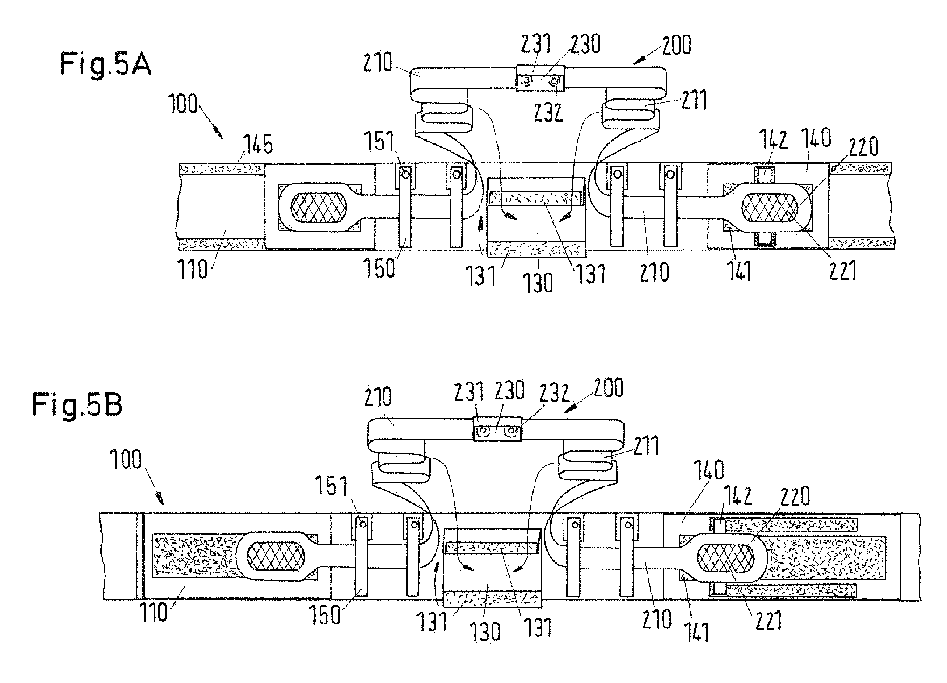

Anhand der Figuren 5A und 5B wird jeweils ein Ausschnitt des Fixiergurtes 100 im Bereich zwischen den Haltevorrichtungen 140 für das Einlegeband 200 dargestellt. Die nicht entfalteten Bereiche 211 des Einlegebandes 200 sind außerhalb der Tasche 130 angeordnet. Au-βerdem wird durch die Fig. 5A und 5B veranschaulicht, dass die nicht entfalteten Bereiche 211 des Einlegebandes 200 bei beiden Ausführungsformen der Erfindung zusammen mit der Tasche 230 für die anschließbare Sende- und/oder Steuereinheit in der Tasche 130 verstaubar sind , wobei verschiedenste Faltungsvarianten möglich sind. Die Tasche 130 wird im Anschluss mittels der Klettverschlüsse 131 so geschlossen, dass die eingelegten Bandbereiche 211 stabil darin verbleiben. Die Anordnung der diagnostischen Sensoren 220 an den Haltevorrichtungen 140 des Fixiergurts 100 entspricht jeweils der Darstellung aus Fig. 4A bzw. 4 B.

Die Fig. 6A und 6B zeigen jeweils einen Ausschnitt des Fixiergurtes 100 im Bereich zwischen den Haltevorrichtungen 140 für das Einlegeband 200 mit teilweise angebrachtem Einlegeband 200 aus den Fig. 5A bzw. 5B. Dargestellt ist jeweils die tierabgewandte Rückseite des rechts gezeigten diagnostischen Sensors 220 (rechter diagnostischer Sensor 220) mit aufgeklapptem Halteriemen 142, welcher selbst mit Klettverschlüssen 143 anbringbar ist. Der rechte diagnostische Sensor 220 weist jeweils auf seiner Rückseite eine Halteschlaufe 222 auf, durch welche der Halteriemen 142 durchführbar ist. Auf der links gezeigten Seite ist jeweils eine weitere Befestigungsmöglichkeit der diagnostischen Sensoren 220 dargestellt, bei der die diagnostischen Sensoren durch Klettverschluss an der links gezeigten Haltevorrichtung 140 des Fixiergurts 100 fixierbar sind. Auf der jeweils linken Seite der Fig. 6A und 6B ist außerdem angedeutet, dass unter den Haltevorrichtungen 140 Unterlegmaterialien, wie hier beispielsweise ein Schwamm oder ein Stück Schaumstoff 400 zur Verstärkung des Anpressdrucks der diagnostischen Sensoren 220, einführbar sind. Durch Erhöhung des Anpressdrucks der diagnostischen Sensoren 220 wird die Messgenauigkeit insbesondere bei behaarten Stellen gesteigert, da der Kontakt der Sensoren mit der Haut des Tieres verbessert wird.

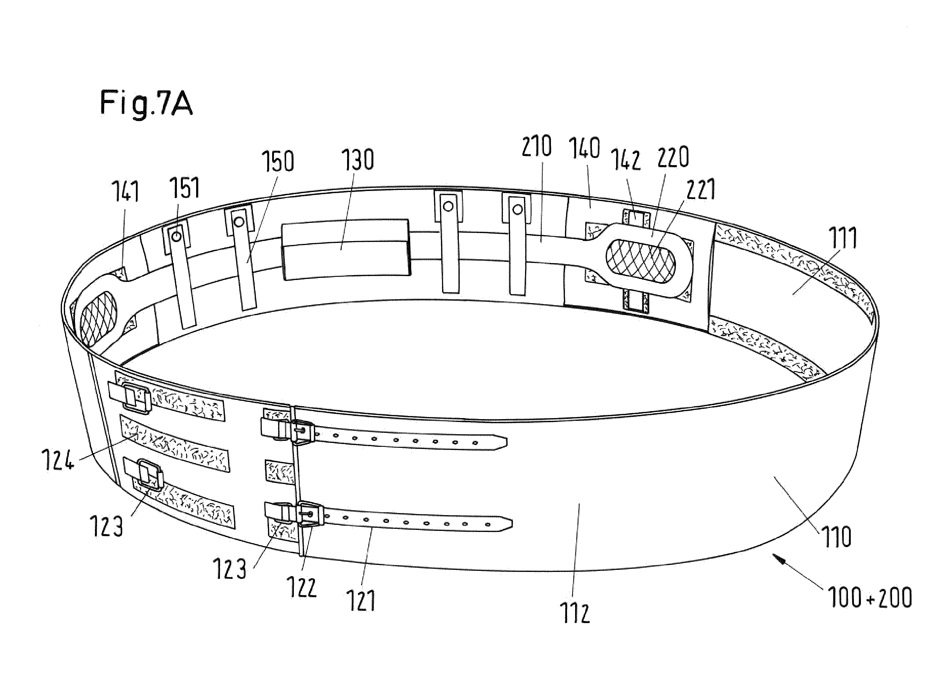

Fig. 7A zeigt den Fixiergurt 100 nach der ersten Ausführungsform der Erfindung im geschlossenen Zustand mit an den Haltevorrichtungen 140 angebrachtem Einlegeband 200 mit den diagnostischen Sensoren 220.

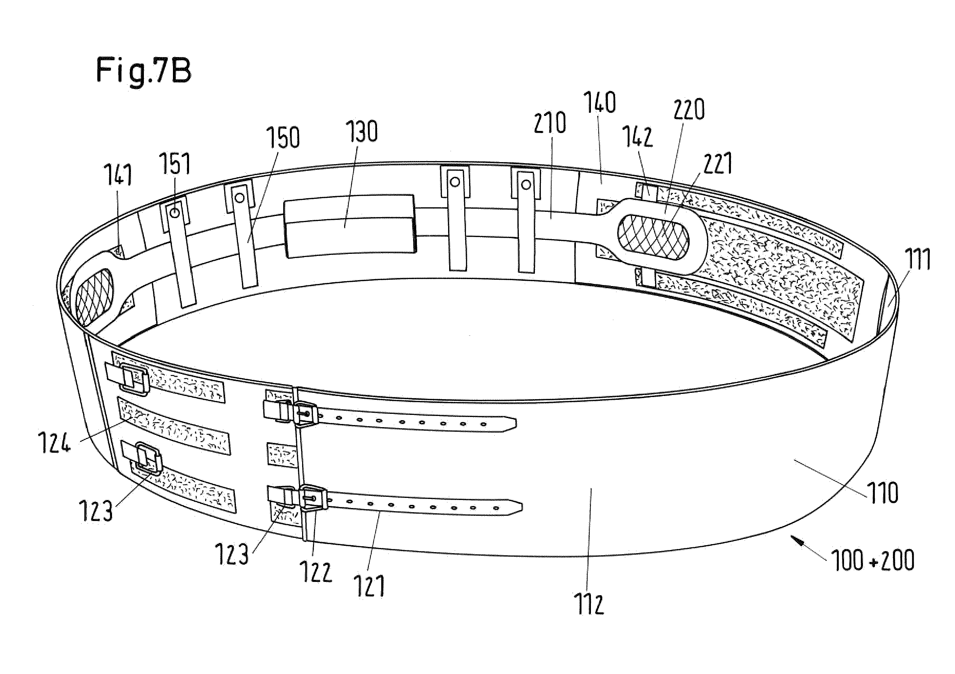

Fig. 7B zeigt den Fixiergurt 100 nach der zweiten Ausführungsform der Erfindung im geschlossenen Zustand mit an den Haltevorrichtungen 140 angebrachtem Einlegeband 200 mit den diagnostischen Sensoren 220.

Die Fig. 2, 4A, 4B, 5A, 5B, 6A, 6B, 7A und 7B beziehen sich, ohne dass darauf in den vorangegangenen Absätzen explizit Bezug genommen wurde nicht nur auf einen Fixiergurt für die veterinärmedizinische Diagnostik bei Großtieren, sondern auch auf einen veterinärmedizinischen Messgürtel zur Messung der Herzfrequenzvariabilität von Großtieren.

Die beiden Ausführungsformen der Innenseite des Fixiergurts sind grundsätzlich auch miteinander kombinierbar. So kann beispielsweise eine variabel anheftbare Haltevorrichtung eine Haltevorrichtung für ein variables Anheften eines diagnostischen Sensors/einer Elektrode aufnehmen. Außerdem kann vorgesehen sein, dass eine Haltevorrichtung des Fixiergurts nach der ersten Ausführungsform ausgelegt ist, während die andere Haltevorrichtung nach der zweiten Ausführungsform vorgesehen ist.

### Bezugszeichenliste

| | |
|---|---|
| 100 | Fixiergurt |
| 110 | Gurt |
| 111 | Innenseite des Fixiergurts |
| 112 | Außenseite des Fixiergurts |
| 120 | Schließvorrichtung/Riemen des Fixiergurts |
| 121 | Riemengurt |
| 122 | Riemenschnalle |
| 123 | Riemenumlenkschnalle |
| 124 | Klettband (Haken- oder Pilzband) |
| 125 | Klettband (Velours- oder Flauschband) |
| 130 | in Längsrichtung offene Tasche für eine anschließbare Steuer- und/oder Sendeeinheit |
| 131 | Klettverschluss der in Längsrichtung offenen Tasche |
| 140 | Haltevorrichtungen für diagnostische Sensoren |
| 141 | Klettband der Haltevorrichtungen für diagnostische Sensoren |
| 142 | Halteriemen für diagnostische Sensoren |
| 143 | Klettverschluss für den Halteriemen für diagnostische Sensoren |
| 144 | Einschubtasche der Haltevorrichtung |
| 145 | Klettband für Haltevorrichtungen |
| 150 | Halteriemen für das Einlegeband |
| 151 | Druckknöpfe für die Halteriemen |
| 200 | Einlegeband |
| 210 | Bandbereich des Einlegebandes |
| 211 | nicht entfaltete Bandbereiche |
| 220 | Diagnostischer Sensor/Elektrode |
| 221 | Meßbereich des diagnostischen Sensors |
| 222 | Halteschlaufe des diagnostischen Sensors/Elektrode |
| 230 | Tasche des Einlegebandes für eine anschließbare Steuer- und/oder Sendeeinheit |
| 231 | Klettverschluss der Tasche des Einlegebandes |
| 232 | Anschlüsse/Druckknöpfe für eine anschließbare Steuer- und/oder Sendeeinheit |
| 300 | Gurt zur Messung der Herzfrequenzvariabilität gemäß dem Stand der Technik |
| 310 | Innenseite des Messgurtes |
| 311 | Außenseite des Messgurtes |
| 312 | Flexibler Bereich des Messgurtes |
| 313 | Rigider Bereiche des Messgurtes |
| 314 | Verlängerungseinrichtung des flexiblen Bereiches |
| 320 | Steckverschluss des Messgurtes |
| 330 | Tasche des Messgurtes für eine anschließbare Steuer- und/oder Sendeeinheit |
| 331 | Klettverschluss der Tasche des Messgurtes |
| 332 | Anschlüsse/Druckknöpfe für eine anschließbare Steuer- und/oder Sendeeinheit |
| 340 | Elektrodenbereiche des Messgurtes |
| 400 | Schwamm/Schaumstoff |

## Patentansprüche

1. Fixiergurt (100) für die veterinärmedizinische Diagnostik bei Großtieren mit einer Innenseite (111) und einer Außenseite (112), wobei die Innenseite (111) im Gebrauchszustand auf dem Fell des zu untersuchenden Großtieres aufliegt, mit einer langgestreckten Ausdehnung in einer Längsrichtung und einer Breite in einer Querrichtung, einen Gurt (110) aus reißfestem Material und mindestens eine Schließvorrichtung (120) umfassend, **dadurch gekennzeichnet, dass**
außerdem an der Innenseite (111) eine oder mehrere Haltevorrichtungen (140) für mindestens zwei diagnostische Sensoren (220) vorgesehen sind, welche geeignet ist bzw. sind, die diagnostische Sensoren (220) in einem variabel einstellbaren Abstand zueinander aufzunehmen.

2. Fixiergurt (100) nach Anspruch 1, **dadurch gekennzeichnet, dass** das Material des Gurtes (110) zumindest im Bereich zwischen den diagnostischen Sensoren elastisch ist.

3. Fixiergurt (100) nach Anspruch 1 oder 2, **dadurch gekennzeichnet, dass** die Haltevorrichtungen (140) geeignet sind, handelsübliche diagnostischen Sensoren (220) aufzunehmen, wobei die diagnostischen Sensoren (220) vorzugsweise an einem Einlegeband (200) angeordnet sind, in dem eine elektrisch leitende Verbindung zwischen den Sensoren (220) und eine Tasche (230) für eine anschließbare Steuer- und/oder Sendeeinheit eingearbeitet sind und wobei das Einlegeband (200) besonders vorzugsweise eine Breite aufweist, die geringer als die Breite des Fixiergurtes (100) ist.

4. Fixiergurt (100) nach Anspruch 3, **dadurch gekennzeichnet, dass** der Fixiergurt (100) an seiner Innenseite innerhalb des Bereichs der Haltevorrichtung(en) zusätzlich mindestens eine in Längsrichtung offene Tasche (130) aufweist, in welche vorzugsweise neben der Tasche (230) des Einlegebandes (200) mit der Steuer- und/oder Sendeeinheit für die veterinärmedizinische Diagnostik auch nicht entfaltete Bandbereiche (211) des Einlegebandes (200) aufnehmbar sind.

5. Fixiergurt (100) nach einem der vorangegangenen Ansprüche, **dadurch gekennzeichnet, dass** als Haltevorrichtungen (140) Klettverschlüsse (141), Halteriemen (142) und/oder Druckknopfverschlüsse vorgesehen sind.

6. Fixiergurt (100) nach einem der vorgegangenen Ansprüche, **dadurch gekennzeichnet, dass** die Schließvorrichtung (120) des Fixiergurts (100) Klettverschlüsse (124, 125) und/oder Riemen (121) und Schnallen (122, 123) umfasst.

7. Fixiergurt (100) nach einem der vorgegangenen Ansprüche, **dadurch gekennzeichnet, dass** die diagnostischen Sensoren (220) als Elektroden ausgestaltet sind, die vorzugsweise für eine Herzfrequenzvariabilitätsmessung geeignet sind.

8. Fixiergurt (100) nach einem der vorgegangenen Ansprüche, **dadurch gekennzeichnet, dass** der Fixiergurt (100) zum Umschnallen um Bauch und Widerrist/Rücken des Großtieres ausgelegt ist.

9. Fixiergurt (100) nach einem der vorgegangenen Ansprüche, **dadurch gekennzeichnet, dass** der Fixiergurt (100) an seiner Außenseite (112) eine weitere Tasche für den Empfänger/Uhr aufweist.

10. Veterinärmedizinischer Messgürtel (100+200) zur Messung der Herzfrequenzvariabilität von Großtieren mit einer Innenseite (111) und einer Außenseite (112), wobei die Innenseite (111) im Gebrauchszustand auf dem Fell des zu untersuchenden Großtieres aufliegt, mit einer langgestreckten Ausdehnung in einer Längsrichtung und einer Breite in einer Querrichtung, einen Gurt (110) aus reißfesten Material, mindestens eine Schließvorrichtung (120) und ein Einlegeband (200) mit mindestens zwei diagnostischen Sensoren (220) umfassend, wobei die diagnostischen Sensoren (220) des Einlegebandes (200) elektrisch leitend im Einlegeband (200) miteinander verbunden sind und zwischen den Sensoren (220) eine Tasche (230) für eine anschließbare Steuer- und/oder Sendeeinheit eingearbeitet ist, **dadurch gekennzeichnet, dass** außerdem an der Innenseite (111) eine oder mehrere Haltevorrichtungen (140) für die diagnostische Sensoren (220) des Einlegebandes (200) vorgesehen sind, welche geeignet sind, die diagnostische Sensoren (220) in einem variabel einstellbaren Abstand zueinander aufzunehmen.
